## Europäisches Patentamt
## European Patent Office
## Office européen des brevets

(19)

(11) Publication number: **0 156 049**
A1

# (12) EUROPEAN PATENT APPLICATION

(21) Application number: **84301835.9**

(22) Date of filing: **19.03.84**

(51) Int. Cl.⁴: **A 61 B 5/08**
**G 01 N 33/497**

(43) Date of publication of application:
**02.10.85 Bulletin 85/40**

(84) Designated Contracting States:
**AT BE CH DE FR GB IT LI LU NL SE**

(71) Applicant: **TRUPHARM LTD**
**Beit Merkazim Maskit Street**
**Herzlia 46733(IL)**

(72) Inventor: **Podoshin, Ludwik, Prof.**
**P.O. Box 6016**
**Haifa(IL)**

(72) Inventor: **Gruenwald, Theodor, Dr.**
**68 Horeb St.**
**Haifa(IL)**

(72) Inventor: **Gertner, Rachel, Dr.**
**25 Burla St.**
**Haifa(IL)**

(74) Representative: **Knott, Stephen Gilbert et al,**
**MATHISEN, MACARA & CO. The Coach House 6-8**
**Swakeleys Road**
**Ickenham Uxbridge UB10 8BZ(GB)**

(54) Device for diagnosing nasal obstructions.

(57) A diagnostic device for checking for obstruction of the nasal airways comprises a sheet-like member having one substantially flat surface adapted to produce at least temporarily visible traces when expired upon through the nostrils. The surface is provided with a plurality of markings against which at least one dimension of the traces can be measured. The surface of the member may be treated with a substance capable of chemically reacting with at least one of the components of the expiratory air, to the effect of changing the color of the expiratory-air-impacted portions of the surface.

FIG.3

EP 0 156 049 A1

0156049

## DEVICE FOR DIAGNOSING NASAL OBSTRUCTIONS

The present invention relates to a diagnostic device for checking for obstruction of the nasal airways.

Nasal obstructions, both congenital and acquired, e.g., post-traumatic, can largely be alleviated by corrective surgery, which must obviously be preceded by a diagnostic procedure establishing the existence, and determining the degree of, such an obstruction. Such procedures have long been known, using spirometric and rhinomanometric methods. Uddströmer (1940), for instance, in order to accomplish a spirometric measurement, used a face mask which was divided into a separate compartment for each nostril. Rhinomanometry, as we now know it, was first employed by Courtade (1903), Seebohm and Hamilton (1958) introduced the method of posterior rhinomanometry. In 1976, Mercury Electronics developed the NRG Nasal Resistance Meter, this instrument measures nasal air flow, using a special face-mask incorporating a linear pneumotachograph and a mouth-piece, the latter allowing the intrapharyngeal pressure to be measured via a small tube passed orally into the pharynx. Mygind et al. (1977) found that 17 per cent of patients were unable to relax the soft palate by this method. Foxen et al. (1971) found that 25 per cent of patients could not be examined by this method for the same reason.

Cottle (1968) described a method of rhino-sphygmomanometry, according to which the air flow was measured via a nozzle on one side of the nose, and the pressure was recorded on the other side.

- 2 -

Tucker (1977) has shown that such a method of measurement may cause a distortion of the nostril, altering the relationships between the lower lateral and upper lateral cartilages and the nasal septum, which can effect the resistance to air flow. In spite of the large numbers of functional tests and modifications that have been reported, the ideal method has yet to be found.

The state of rhinomanometry today is comparable to that of audiometry 40 years ago. There are many methods of measurement that one can use and many people interested in this field have designed techniques, none of which has achieved universal acceptance.

It is one of the objects of the present invention to overcome the drawbacks and disadvantages of the prior-art devices used for the above-mentioned purpose, and to provide a device that will interfere neither with the anatomy of the nose, nor with physiological air flow and pressure, that enables tests to be performed easily, rapidly and inexpensively, and with no discomfort to the subject, and that yields reproducible results.

This the present invention achieves by providing a diagnostic device for checking for obstruction of the nasal airways, comprising a sheet-like member having at least one substantially flat surface adapted to produce at least temporarily visible traces when expired upon through the nostrils, wherein said surface is provided with a plurality of markings against which at least one dimension of said traces can be measured.

The invention will now be described in connection with certain preferred embodiments with reference to the following illustrative figures so that it may be more fully understood.

With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

Fig. 1 is a top view, to a reduced scale, of the device according to the invention;

Fig. 2 shows the result of a test performed on a subject free of nasal pathology, and

Figs. 3 to 6 present the results of tests on subjects with nasal obstructions of various types and degrees of severity.

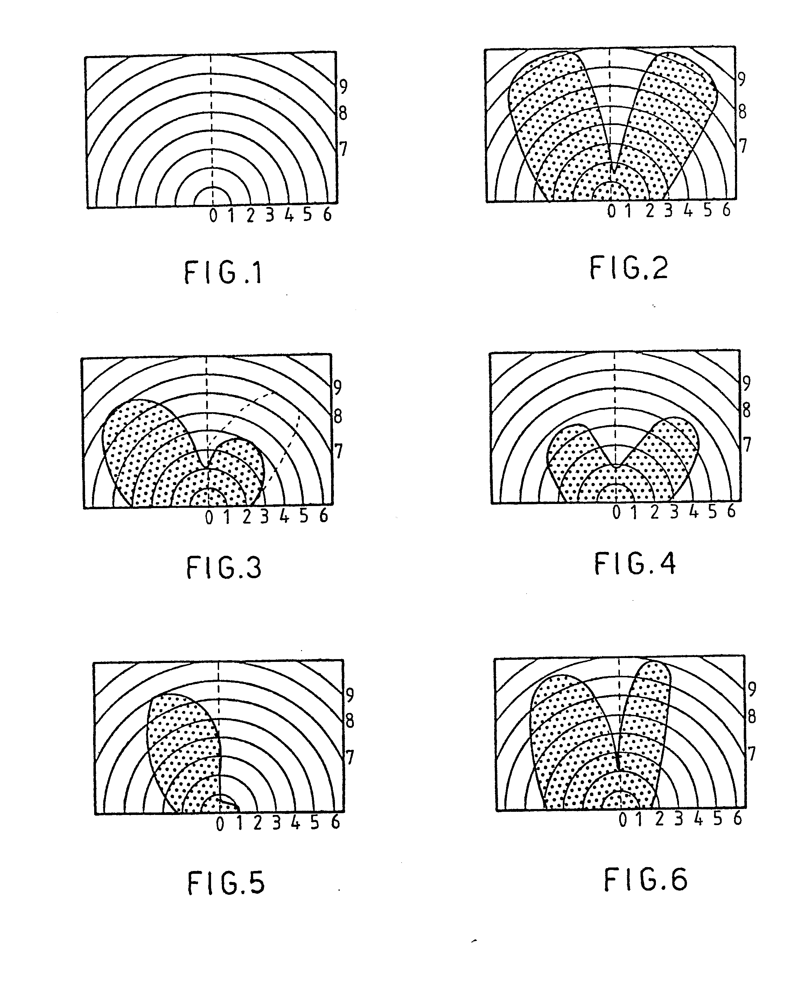

Referring now to the drawings, there is seen in Fig. 1 a top view of the device according to the invention. In a preferred embodiment, the device consists of a substrate made of a relatively stiff cardboard, to which is bonded, as top surface, a glossy, mirror-like piece of aluminum foil. On its glossy surface, this foil carries markings in the form of concentric arcs numbered 1 to 9, the radius of the smallest arc (No. 1) being 1 cm, and the radii of the other arcs increasing at the rate of 1 cm per radius. Each number thus signifies the radius of its respective arc in cm. Starting from the point of origin, 0, a dashed line crosses all arcs at a right angle to the base line.

In use, the card, mirror side up, is horizontally held under the subject's nostrils, with the point of origin 0 closest to his face and the dashed line below the columnella. The subject is instructed to breathe through the nose slowly, without force, and with closed mouth. When he expires through the nose, the difference in temperature between the expiratory air and the surface of the device on which this air impinges is such as to cause the water vapor in the expiratory air to condense instantaneously and to precipitate on the glossy surface, misting it in the process and thus producing two easily perceived lobe-like shapes which, as will be shown with the aid of further drawings, constitute an indication of the condition of the nasal airways. The misted areas persist for a sufficient length of time for the tester to measure the length of the lobes with the aid of the concentric arcs and, perhaps in a second test, also their maximum width, which has a bearing on a special type of nasal obstruction.

Tests, using the device according to the invention, were performed on a control group of significant size, of subjects free of nasal pathology or any complaints regarding their ability to breathe through the nose, and whose nasal septum was straight and centered, as established by physical examination. A characteristic misting pattern produced by these healthy subjects is shown in Fig. 2. For the whole group, the length of the lobes varied between 7 and 8 cm, and their width between 4 and 5 cm.

Characteristic patterns obtained from a group of patients with deformed or deviated septa are shown in the following figures, in which Fig. 3 illustrates reduced breathing through the right nostril in a patient with a deviated septum, the lefthand lobe being 7 cm long, the righthand lobe, 4 cm. In Fig. 4 both lobes are of reduced length. This type of pattern was produced by a patient with an S-shaped deformation of the nasal septum. Fig. 5 illustrates a completely blocked righthand side of the nose, caused by a post-traumatic deviation of the nasal septum. A particular pattern is seen in Fig. 6, in which a special type of nasal obstruction is indicated by the lobe on the right, which, although even somewhat longer than the left lobe, is only about half as wide. In such cases diagnosis is often helped by calculating the area covered by the lobe in question, which is nearly elliptical in shape and is given by the equation $A = \pi.a.b.$, in which $a$ is the length of the lobe as read off the arcs, and b is its maximum width.

For the convenience of the examining physician, reduced reproductions of these archetypal cases are advantageously printed on the back side of the device.

For protection of the glossy surface, the device is advantageously provided with lateral flaps that fold towards the center and cover the surface when the device is not in use.

While the combination: cardboard substrate/metal foil was found to be most efficient as well as inexpensive, it is of course also possible to use other substrates and mirror-like surfaces, such as a chromium-plated metal sheet, or a metallized plastic card, or the like.

Permanent or near-permanent records of these patterns may be obtained with the aid of another embodiment of the invention, in which at least the surface of the device is treated with a substance capable of chemically reacting with at least one of the components of the expiratory air, to the effect of changing the color of that portion of the surface that is impacted by this air.

Thus while the hereinbefore described embodiments are based on condensation of inhaled air which is heated and humidified in the nasal passages before expulsion and then temporarily fogs a cooler surface, surfaces which undergo prolonged and/or permanent change when exposed to moisture could also be used, e.g., by impregnating the surface of the device with a chemical which undergoes color change in the presence of moisture (c.f. U.S. Patent 4,163,427) or with a combination of chemicals which undergo chemical reaction and color change in the presence of moisture (e.g., a dry base or acid and litmus indicator).

Preferably the surface could be impregnated with a combination of chemicals which undergo color change in the presence of $CO_2$ and moisture such as Whatman filter paper no. 1, 2 or 3 impregnated with a solution having the following components:

a) A _solvent_ comprising a mixture of lower alcohol such as methanol or ethanol with water. The alcohol is provided to render better solubility to the organic ingredients in the impregnating solution and its content in the mixture should be about 10-50% by volume ($^V$/v);

b) An <u>alkali</u> for the absorption and reaction with carbon dioxide which alkali may ᵤₑ a mixture of an organic amine such as ethylene diamine, ᵤₜₕₐnolamine or butylamine with an inorganic alkali hydroxᵢᵤₑ as LiOH, NaOH or KOH.

The amine assures a rapid ᵤₗsorption of carbon dioxide while the hydroxide content rendₑrₛ to the solution the necessary pH of 10-11 and also ᵢ good and stable color to the acid-base indicator added.

The concentrations of the amine and the hydroxide in the impregnating solution may range from about 5-25% $^V$/v and 0.01-0.05 M respectively.

c) <u>alkaline-earth soluble salt</u> such as chlorides or nitrates of calcium, barium or strontium may be used for rendering irreversibility to the reaction of carbon dioxide with the alkali by forming insoluble carbonates. The mole ratio of the salt to the hydroxide in the impregnating solution may be about 1:2.

d) An <u>indicator</u> which changes its color upon the reaction of carbon dioxide with the impregnated paper, can be any acid-base indicator, or a mixture of them, with a pK between 8.5 and 10.2, such as Thymol blue, Cresol red or Thumolphthalein, and should have a concentration of at least 0.01% $^W$/v of the alcohol content in the impregnating solution.

e) A <u>humidifier</u> that assures a minimum water content in the finished air dried impregnated paper which facilitates the diffusion of the carbon dioxide gas to and reaction with, the chemicals in the paper. The best suitable for this purpose is glycerine in a concentration of 2-6% $^W$/v in the impregnating solution.

- 8 -

The following illustrative example will serve to illustrate the practice of the above described embodiments.

Example

Whatman No. 1 filter paper marked with a plurality of concentric arcs is impregnated with a 0.02 M LiOH solution containing: 30% $^V$/v Ethanol; 15% $^V$/v 2-Aminoethanol; 0.01 M $BaCl_2$; 0.2% $^W$/v of O-cresolphthalein-thymol blue indicator mixture in the ratio 4:1 respectively, and 5% $^V$/v glycerine.

It will be evident to those skilled in the art that the invention is not limited to the details of the foregoing illustrative embodiments and example and that the present invention may be embodied in other specific forms without departing from the essential attributes thereof, and it is, therefore, desired that the present embodiments and example be considered in all respects as illustrative and not restrictive, reference being made to the appended claims, rather than to the foregoing description, and all changes which come with the meaning and range of equivalency of the claims are, therefore, intended to be embraced therein.

## CLAIMS

1. A diagnostic device for checking for obstruction of the nasal airways, characterised in that the device comprises a sheet-like member having at least one substantially flat surface adapted to produce at least temporarily visible traces when expired upon through the nostrils, and in that said surface is provided with a plurality of markings (1-9) against which at least one dimension of said traces can be measured.

2. A device as claimed in claim 1, characterised in that said markings (1-9) are in the form of a plurality of concentric arcs.

3. A device as claimed in claim 1 or 2, characterised in that said surface is a mirror-like surface upon which the water vapor contained in the expiratory air condenses, forming said traces.

4. A device as claimed in any one of the preceding claims, characterised in that said surface comprises a glossy metal foil mounted on a cardboard substrate.

5. A device as claimed in claim 1 or 2, characterised in that at least the surface of said member is treated with a substance capable of chemically reacting with at least one of the components of the expiratory air, to the effect of changing the color of the expiratory-air-impacted portions of said surface.

6. A device as claimed in claim 5, characterised in that the surface of said member is treated with a plurality of chemicals effecting a color change in the presence of exhaled carbon dioxide.

7. A device for checking the obstruction of the nasal airways characterised in that the device comprises a member having a flat surface adapted to be disposed under the nostrils, in use, said surface being such that exhaled air from the nostrils leaves a visible mark thereon.

FIG.1

FIG.2

FIG.3

FIG.4

FIG.5

FIG.6

0156049

Application number

EP 84 30 1835

European Patent Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | FR-A- 902 661 (C. ROGUE) * whole document * | 1-4,7 | A 61 B 5/08 G 01 N 33/497 |
| A | US-A-2 795 223 (G.K.E.H. STAMPE) * column 2, lines 24-34; figures 1-3 * | 5,6 | |
| A | US-A-3 420 224 (A.F. FARR) * column 3, lines 44-52, 65-75; column 4, lines 60-71; column 5, lines 1-8; figures 2,6 * | 5,6 | |
| A | US-A-3 507 269 (H.H. BERRY) * column 2, lines 24-35; column 3, lines 53-59, 69-73; figures 1-3 * | 4-6 | |

TECHNICAL FIELDS SEARCHED (Int. Cl.4)

A 61 B
G 01 N

The present search report has been drawn up for all claims

| Place of search | Date of completion of the search | Examiner |
|---|---|---|
| THE HAGUE | 19-11-1984 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document